(19) Europäisches Patentamt
European Patent Office
Office européen des brevets

(11) EP 0 876 136 B1

(12) EUROPÄISCHE PATENTSCHRIFT

(45) Veröffentlichungstag und Bekanntmachung des Hinweises auf die Patenterteilung:
**08.05.2002 Patentblatt 2002/19**

(21) Anmeldenummer: **97901545.0**

(22) Anmeldetag: **17.01.1997**

(51) Int Cl.$^7$: **A61K 7/42**

(86) Internationale Anmeldenummer:
**PCT/EP97/00217**

(87) Internationale Veröffentlichungsnummer:
**WO 97/26857 (31.07.1997 Gazette 1997/33)**

# (54) STABILE KOSMETISCHE UND DERMATOLOGISCHE LICHTSCHUTZZUBEREITUNGEN IN FORM VON W/O-EMULSIONEN MIT EINEM GEHALT AN ANORGANISCHEN MIKROPIGMENTEN, TRIAZINDERIVATEN UND/ODER WEITEREN KOMPONENTEN

STABLE COSMETIC AND DERMATOLOGICAL LIGHT-PROTECTING PREPARATIONS IN THE FORM OF WATER/OIL EMULSIONS CONTAINING INORGANIC MICRO-PIGMENTS, DERIVATIVES OF TRIAZINE AND/OR OTHER COMPONENTS

PREPARATIONS COSMETIQUES ET DERMATOLOGIQUES PHOTOPROTECTRICES STABLES SOUS FORME D'EMULSIONS EAU/HUILE CONTENANT DES MICROPIGMENTS INORGANIQUES, DES DERIVES DE LA TRIAZINE ET/OU D'AUTRES COMPOSANTS

(84) Benannte Vertragsstaaten:
**AT BE CH DE ES FR GB IT LI NL SE**

(30) Priorität: **25.01.1996 DE 19602619**

(43) Veröffentlichungstag der Anmeldung:
**11.11.1998 Patentblatt 1998/46**

(73) Patentinhaber: **Beiersdorf Aktiengesellschaft
20245 Hamburg (DE)**

(72) Erfinder:
- **GERS-BARLAG, Heinrich
D-22527 Hamburg (DE)**
- **DÖRSCHNER, Albrecht
D-22457 Hamburg (DE)**
- **KRÖPKE, Rainer
D-22527 Hamburg (DE)**
- **MÜLLER, Anja
D-20253 Hamburg (DE)**
- **NISSEN, Bente
D-20253 Hamburg (DE)**
- **SCHOMANN, Arianne
D-22391 Hamburg (DE)**

(56) Entgegenhaltungen:
**EP-A- 0 457 687** **US-A- 4 454 113**

- **SEIFEN, OLE, FETTE, WACHSE, Bd. 115, Nr. 18, 1989, Seiten 661-662, XP000081197 K. SPERLING: "UV-Filter für Haut- und Produktschutz in kosmetischen Formulierungen"**
- **DATABASE WPI Week 9233 Derwent Publications Ltd., London, GB; AN 92-272108 XP002029893 "Emulsified cosmetic material for skin, hair or external use medical prods. - contg. lipophilic polyglycerine condensed hydroxy acid ester, amphoteric solvent, lipid and water" & JP 04 178 316 A (KAO) , 25.Juni 1992 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich. ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Ein vorteilhafter UVB-Filter ist der 4,4',4"-(1,3,5-Triazin-2,4,6-triyltriimino)-trisbenzoesäure-tris(2-ethylhexylester), synonym: 2,4,6-Tris-[anilino-(p-carbo-2'-ethyl-1'hexyloxy)]-1,3,5-triazin.

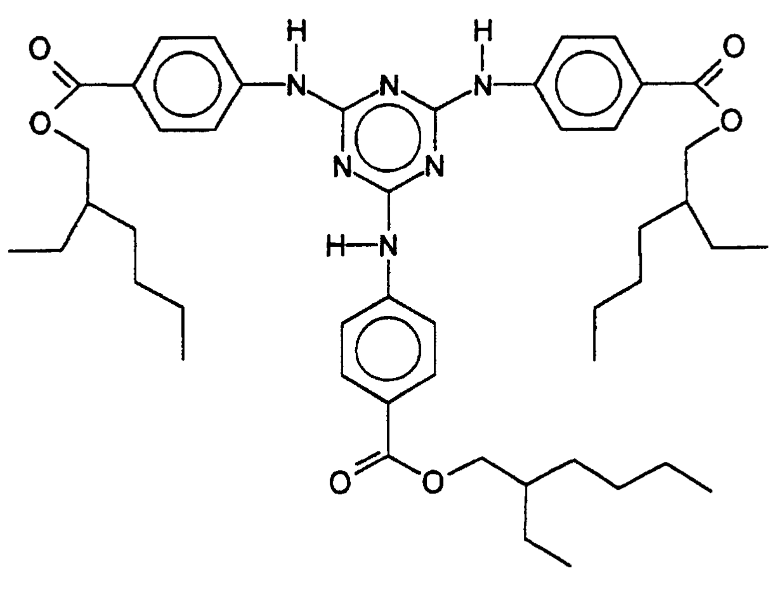

Diese UVB-Fittersubstanz wird von der BASF Aktiengesellschaft unter der Warenbezeichnung UVINUL® T 150 vertrieben und zeichnet sich durch gute UV-Absorptionseigenschaften aus.

Der Hauptnachteil dieses UVB-Filters ist die schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit aktiver, UV-Filtersubstanz.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Die anorganischen Pigmente zeichnen sich an sich durch gute Lichtschutzwirkung aus. Sie haben jedoch den Nachteil, daß es schwierig ist, sie in befriedigender Weise solchen Formulierungen einzuverleiben. Nur wenn die Partikel in der endgültigen Formulierung sehr klein sind, werden sie nach dem Auftragen auf die Haut nicht als störendes "Weißein" (Bildung von weißen Flecken auf der Haut) beobachtet. Üblicherweise liegen die Partikelgrößen solcher Pigmente im Bereich unter 100 nm. In einer herkömmlichen Emulsion neigen die Partikel mehr oder weniger stark zur Zusammenlagerung zu Agglomeraten, welche bereits unter dem Lichtmikroskop zu erkennen sind. Solche Agglomeration ist ferner nicht mit dem Herstellungsprozeß einer entsprechenden Zubereitung abgeschlossen, sondern setzt sich während der Lagerung fort. Das "Weißeln" kann sich daher über einen längeren Zeitraum noch verstärken. Auch kann das Ausölen oder gar Brechen einer Emulsion mittel- oder langfristig Folge einer derartigen Agglomeration sein.

Ein weiterer Nachteil des Einsatzes anorganischer Pigmente in kosmetischen Formulierungen ist, daß solche Pigmente in den weitaus meisten Fällen zu starker Hauttrockenheit führen.

Weiterhin sind bekannte und gebräuchliche Lichtschutzfiltersubstanzen das 4-(tert.-Butyl)-4'-methoxydibenzoylmethan, welches sich durch die Struktur

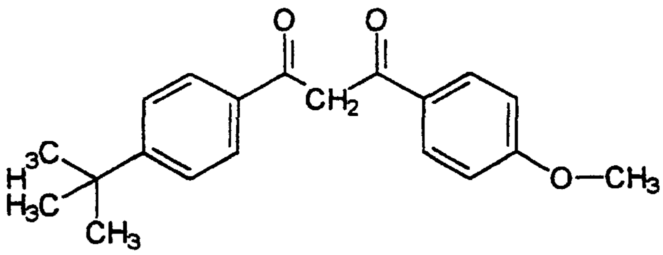

auszeichnet, und von Givaudan unter der Marke Parsol® 1789 verkauft wird, ferner der 4-Methylbenzylidencampher, welcher sich durch die Struktur

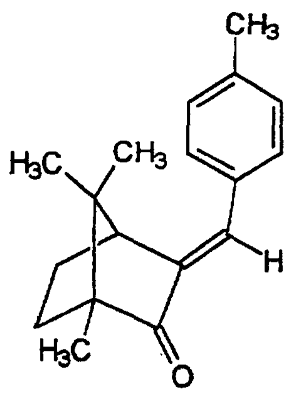

auszeichnet und von Merck unter der Marke Eusolex® 6300 verkauft wird. Diese Substanzen zeichnen sich an sich durch gute UV-Filtereigenschaften aus. Gerade in Kombination miteinander oder anderen als Festkörper vorliegenden Substanzen ist ihre Einsatzkonzentration jedoch begrenzt.

Insbesondere wenn mehrere der unter Normalbedingungen als Festkörper vorliegenden Lichtschutzsubstanzen vorliegen, beispielsweise gewählt aus der Gruppe 4,4',4"-(1,3,5-Triazin-2,4,6-triyltriimino)-tris-benzoesäure-tris (2-ethylhexylester),4-Methylbenzylidencampher, 4-(tert.-Butyl)-4'-methoxydibenzoylmethan und Titandioxid, sind gemäß den Lehren des Standes der Technik nur jeweils geringe Einsatzkonzentrationen und damit niedrige Lichtschutzfaktoren möglich, es sei denn, der Anteil der Ölphase würde überproportional erhöht, was aber ebenfalls Nachteile hätte.

Jedenfalls bestand der Nachteil des Standes der Technik, daß in der Regel entweder nur vergleichsweise niedrige Lichtschutzfaktoren erreicht werden konnten, oder daß die Lichtschutzfilter nicht die genügende UV-Stabilität aufwiesen oder nicht genügende physiologische Verträglichkeit aufwiesen oder nicht genügend hohe Löslichkeit oder Dispergierbarkeit in kosmetischen oder dermatologischen Zubereitungen aufwiesen oder auch sonstige Inkompatibilitäten mit kosmetischen oder dermatologischen Zubereitungen oder mehrere Nachteile zugleich.

Wenigstens einigen, wenn nicht allen diesen Nachteilen abzuhelfen, war eine der Aufgaben der vorliegenden

Erfindung.

Es war indes überraschend und für den Fachmann nicht vorauszusehen, daß kosmetische oder dermatologische Lichtschutzzubereitungen in Form von W/O-Emulsionen, enthaltend

(a) eine oder mehrere unter Normalbedingungen als Feststoff vorliegende Lichtschutzfiltersubstanzen
(b) einen oder mehrere W/O-Emulgatoren, gewählt aus der Gruppe der Substanzen der allgemeinen Strukturformel

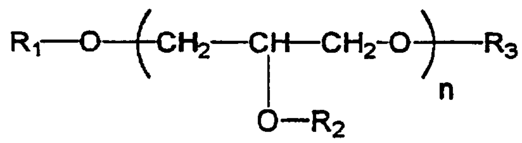

,

wobei $R_1$, $R_2$ und $R_3$ unabhängig voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt.

den Nachteilen des Standes der Technik abhelfen.

Als vorteilhafte Verkörperung der vorliegenden Erfindung werden angesehen: kosmetische oder dermatologische Lichtschutzzubereitungen in Form von W/O-Emulsionen, enthaltend

(a) eine oder mehrere unter Normalbedingungen als Feststoff vorliegende Lichtschutzfiltersubstanzen gewählt aus der Gruppe 4,4',4"-(1,3,5-Triazin-2,4,6-triyltriimino)-tris-benzoesäure-tris(2-ethylhexylester), 4-Methylbenzylidencampher, 4-(tert.-Butyl)-4'-methoxydibenzoylmethan, anorganische Mikropigmente, insbesondere hydrophobierte anorganische Mikropigmente, und
(b) einen oder mehrere W/O-Emulgatoren, gewählt aus der Gruppe der Substanzen der allgemeinen Strukturformel

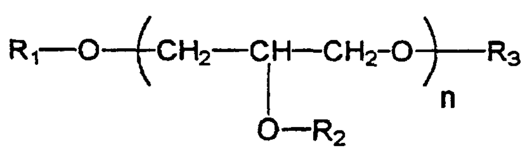

,

wobei $R_1$, $R_2$ und $R_3$ unabhängig voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt.

Als besonders vorteilhafte Verkörperung der vorliegenden Erfindung werden angesehen: kosmetische oder dermatologische Lichtschutzzubereitungen in Form von W/O-Emulsionen, enthaltend

(a) 4,4',4"-(1,3,5-Triazin-2,4,6-triyltriimino)-tris-benzoesäure-tris(2-ethylhexylester), 4-Methylbenzylidencampher, 4-(tert.-Butyl)-4'-methoxydibenzoylmethan und hydrophobiertes Titandioxid als anorganisches Mikropigment, und
(b) einen oder mehrere W/O-Emulgatoren, gewählt aus der Gruppe der Substanzen der allgemeinen Strukturformel

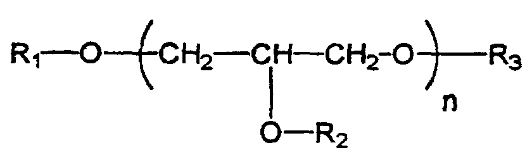

,

wobei $R_1$, $R_2$ und $R_3$ unabhängig voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt.

Als ganz besonders vorteilhaft hat sich der Emulgator "Polyglyceryl-2-Polyhydroxystearat" herausgestellt, welcher unter den Regstriemummem 156531-21-4 bzw. 144470-58-6 in den "Chemical Abstracts" abgelegt ist, und

welcher beispielsweise unter der Warenbezeichnung DEHYMULS® PGPH von der Henkel KGaA erhältlich ist.

Die japanische Offenlegungsschrift JP-Hei-04/178316 beschreibt zwar kosmetische Zubereitungen mit einem Gehalt an Emulgatoren, welche unter die unter (b) aufgeführte Strukturformel fallen, ein Hinweis auf die vorliegende Erfindung wird jedoch a.a.O. nicht gegeben.

In den erfindungsgemäßen Wirkstoffkombinationen bzw. in kosmetischen oder dermatologischen Zubereitungen, diese Wirkstoffkombinationen enthaltend, haben auch die schwererlöslichen Komponenten eine bessere Löslichkeit als in den Zubereitungen des Standes der Technik, auch dann, wenn mehrere solcher Komponenten vorliegen.

Erfindungsgemäß kann ferner die Agglomeration eventuell vorhandener anorganischer Pigmentpartikel (welche natürlich dispergiert, und nicht gelöst, vorliegen) mit den Folgen "Weißeln", Ausölen, Brechen der Emulsion verhindert werden, auch dann, wenn zusätzlich ein oder mehrere schwererlösliche Komponenten vorliegen.

Weiterhin sind erfindungsgemäß Lichtschutzzubereitungen erhältlich, welche höhere Stabilität, insbesondere Stabilität gegen Zersetzung unter dem Einfluß von Licht, ganz besonders UV-Licht, aufweisen, als der Stand der Technik hätte erwarten lassen. Weiterhin sind erfindungsgemäß besonders gut hautverträgliche Zubereitungen erhältlich.

Voraussetzung für die Verwendbarkeit der erfindungsgemäßen Wirkstoffkombinationen für die erfindungsgemäßen Zwecke ist natürlich die kosmetische bzw. dermatologische Unbedenklichkeit der zugrundeliegenden Substanzen.

Es ist erfindungsgemäß möglich die Einsatzmengen von 4,4',4"-(1,3,5-Triazin-2,4,6-triyltriimino)-tris-benzoesäure-tris(2-ethylhexylester), aber auch der anderen unter Normalbedingungen als Feststoff vorliegenden Lichschutzfiltersubstanzen, in kosmetischen oder dermatologischen Zubereitungen gegenüber dem Stande der Technik wesentlich zu erhöhen.

Ferner war erstaunlich, daß durch Zugabe erfindungsgemäß verwendeter Emulgatoren und 4-(tert.-Butyl)-4'-methoxydibenzoylmethan und/oder 4-Methylbenzylidencampher eine Stabilisierung von Lösungen des 4,4', 4"-(1,3,5-Triazin-2,4,6-triyltriimino)-trisbenzoesäure-tris(2-ethylhexylester) bewirkt wird, da die letztere Substanz nicht nur schlechte Löslichkeit aufweist, sondern auch aus seiner Lösung leicht wieder auskristallisiert. Erfindungsgemäß ist daher auch ein Verfahren zur Stabilisierung von Lösungen des 4,4',4"-(1,3,5-Triazin-2,4,6-triyltriimino)-tris-benzoesäure-tris(2-ethylhexylester), dadurch gekennzeichnet, daß solchen Lösungen ein wirksamer Gehalt an einem oder mehreren erfindungsgemäß verwendeten Emulgatoren und 4-(tert.-Butyl)-4'-methoxydibenzoylmethan und/oder 4-Methylbenzylidencampher zugesetzt wird.

Weiterhin war erstaunlich, daß die UV-Stabilität der einzelnen eingesetzten UV-Filtersubstanzen, insbesondere des 4-(tert.-Butyl)-4'-methoxydibenzoylmethans, erfindungsgemäß erheblich erhöht werden kann.

Die Gesamtmenge an 4,4',4"-(1,3,5-Triazin-2,4,6-triyltriimino)-tris-benzoesäure-tris(2-ethylhexylester) in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(tert.-Butyl)-4'-methoxydibenzoylmethan und/oder 4-Methylbenzylidencampher in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an dem oder den erfindungsgemäßen Emulgatoren in den fertigen kosmetischen oder dermatologischen Zubereitungen wird vorteilhaft aus dem Bereich von 0,1 - 25,0 Gew.-%, bevorzugt 0,5 - 6,0 Gew.-% gewählt, bezogen auf das Gesamtgewicht der Zubereitungen

Erfindungsgemäße kosmetische und dermatologische Zubereitungen enthalten außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma

TAYCA erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfind ungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesterylund Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle. Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;

- Alkylbenzoate;
- Silikonöle wie Dimethylpolysiloxane, Diethylpolysiloxane, Diphenylpolysiloxane sowie Mischformen daraus.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder-monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 3-Benzyiidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Sulfonsäure-Derivate des 3-Benzylidencamphers, wie z.B. 4-(2-Oxo-3-bomylidenmethyl)benzolsulfonsäure,2-Methyl-5-(2-oxo-3-bornylidenmethyl)sulfonsäure und deren Salze.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit UVA-Filtern zu kombinieren, die bisher üblicherweise in kosmetischen Zubereitungen enthalten sind.

Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Femer ist vorteilhaft, die erfindungsgemäßen Wirkstoffkombinationen mit weiteren UVAund/oder UVB-Filtern zu kombinieren, beispielsweise bestimmten Salicylsäurederivaten wie

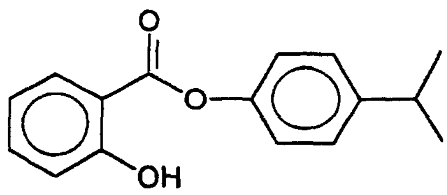

**(4-Isopropylbenzylsalicylat),**

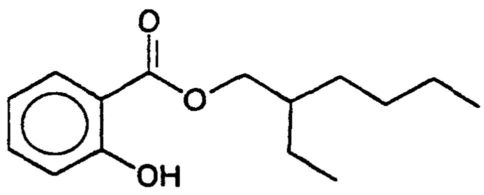

**(2-Ethylhexylsalicylat, Octylsalicylat),**

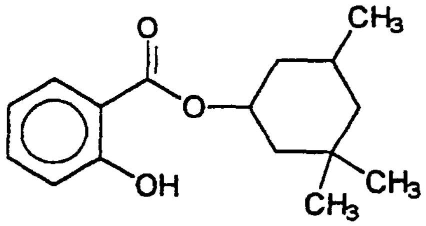

**(Homomenthylsalicylat).**

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1- 10,0 Gew.-%, bevorzugt 0,5 - 5,0 Gew.-% zu wählen.

Das nachfolgende Beispiel soll die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1** | |
|---|---|
| | Gew.-% |
| Glycerylanolat | 1,00 |
| Wollwachsalkohol | 0,10 |
| Polyglyceryl-2 Polyhydroxystearat | 5,00 |
| Paraffinöl (Paraffinum liquidum) | 6,00 |
| isohexadecan | 4,00 |
| Myristylmyristat | 3,00 |
| Butylmethoxydibenzoylmethan | 2,00 |
| Methylbenzylidenecampher | 4,00 |
| Octyltriazon (Uvinul T150) | 1,50 |
| Titandioxid | 2,00 |
| Milchsäure | 1,00 |
| NaOH | q.s. |
| Glycerin | 5,00 |
| Ethanol | 2,00 |
| $MgSO_4$ | 0,70 |

(fortgesetzt)

| Beispiel 1 | |
|---|---|
| | Gew.-% |
| Bisabolol | 0,10 |
| $Na_3HEDTA$ | 0,50 |
| Tocopherylacetat | 0,50 |
| Wasser | ad 100,00 |

## Patentansprüche

**1.** Kosmetische oder dermatologische Lichtschutzzubereitungen in Form von W/O-Emulsionen, enthaltend

(a) eine oder mehrere unter Normalbedingungen als Feststoff vorliegende Lichtschutzfiltersubstanzen
(b) einen oder mehrere W/O-Emulgatoren, gewählt aus der Gruppe der Substanzen der allgemeinen Strukturformel

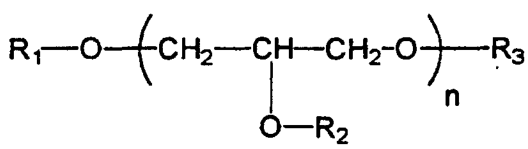

,

wobei $R_1$, $R_2$ und $R_3$ unabhängig voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt.

**2.** Lichtschutzzubereitungen nach Anspruch 1, **dadurch gekennzeichnet, daß** der oder die unter Normalbedingungen als Feststoff vorliegende Lichtschutzfiltersubstanzen gewählt werden aus der Gruppe 4,4',4"-(1,3,5-Triazin-2,4,6-triyltriimino)-tris-benzoesäuretris(2-ethylhexylester), 4-Methylbenzylidencampher, 4-(tert.-Butyl)-4'-methoxydibenzoylmethan, anorganische Mikropigmente, insbesondere hydrophobierte anorganische Mikropigmente.

**3.** Lichtschutzzubereitungen nach Anspruch 1, **dadurch gekennzeichnet, daß** der oder die unter Normalbedingungen als Feststoff vorliegende Lichtschutzfiltersubstanzen gewählt werden aus der Gruppe 4,4',4"-(1,3,5-Triazin-2,4,6-triyltriimino)-tris-benzoesäuretris(2-ethylhexylester), 4-Methylbenzylidencampher, 4-(tert.-Butyl)-4'-methoxydibenzoylmethan und hydrophobiertes Titandioxid als anorganisches Mikropigment.

**4.** Lichtschutzzubereitungen nach Anspruch 1, **dadurch gekennzeichnet, daß** als unter Normalbedingungen als Feststoff vorliegende Lichtschutzfiltersubstanzen gleichzeitig gewählt werden 4,4',4"-(1,3,5-Triazin-2,4,6-triyltriimino)-tris-benzoesäure-tris(2-ethylhexylester), 4-Methylbenzylidencampher, 4-(tert.-Butyl)-4'-methoxydibenzoylmethan und hydrophobiertes Titandioxid als anorganisches Mikropigment.

**5.** Lichtschutzzubereitungen nach Anspruch 1, **dadurch gekennzeichnet, daß** als W/O-Emulgator das Polyglyceryl-2-Polyhydroxystearat gewählt wird.

## Claims

**1.** Cosmetic or dermatological light protection formulations in the form of W/O emulsions, comprising

(a) one or more light protection filter substances which are solid under normal conditions and
(b) one or more W/O emulsifiers chosen from the group of substances of the general structural formula

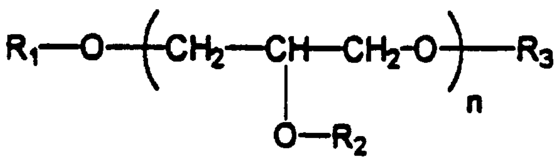

in which $R_1$, $R_2$ and $R_3$ independently of one another are chosen from the group which consists of: H, branched or unbranched, saturated or unsaturated fatty acid radicals having from 8 to 24 carbon atoms, in which up to 3 aliphatic hydrogen atoms may be substituted by hydroxyl groups, and n is a number from 2 to 8.

**2.** Light protection formulations according to Claim 1, **characterized in that** the light protection filter substance(s) which is/are solid under normal conditions is/are chosen from the group consisting of tris(2-ethylhexyl) 4,4',4''-(1,3,5-triazine-2,4,6-triyltriimino)trisbenzoate, 4-methylbenzylidenecamphor, 4-(tert-butyl)-4'-methoxydibenzoylmethane, inorganic micropigments, in particular hydrophobicized inorganic micropigments.

**3.** Light protection formulations according to Claim 1, **characterized in that** the light protection filter substance(s) which is/are solid under normal conditions is/are chosen from the group consisting of tris(2-ethylhexyl) 4,4',4''-(1,3,5-triazine-2,4,6-triyltriimino)trisbenzoate, 4-methylbenzylidenecamphor, 4-(tert-butyl)-4'-methoxydibenzoylmethane and hydrophobicized titanium dioxide as inorganic micropigment.

**4.** Light protection formulations according to Claim 1, **characterized in that** light protection filter substances which are solid under normal conditions which are also selected are tris(2-ethylhexyl) 4,4',4''-(1,3,5-triazine-2,4,6-triyltriimino)trisbenzoate, 4-methylbenzylidenecamphor, 4-(tert-butyl)-4'-methoxydibenzoylmethane and hydrophobicized titanium dioxide as inorganic micropigment.

**5.** Light protection formulations according to Claim 1, **characterized in that** the W/O emulsifier chosen is polyglyceryl-2 polyhydroxystearate.

## Revendications

**1.** Préparations cosmétiques ou dermatologiques photoprotectrices sous la forme d'émulsions E/H, comprenant

(a) une ou plusieurs substances filtrantes photoprotectrices présentes sous forme de solide dans des conditions normales et
(b) un ou plusieurs émulsifiants E/H, choisis parmi le groupe des substances de formule structurale générale

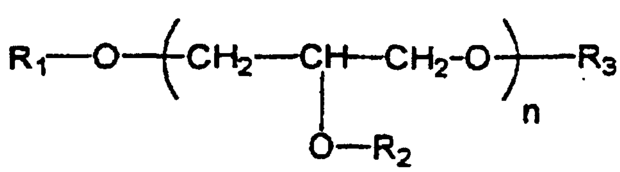

dans laquelle $R_1$, $R_2$ et $R_3$ sont choisis, indépendamment les uns des autres, parmi le groupe comprenant : H, des radicaux acides gras ramifiés ou non ramifiés, saturés ou insaturés, ayant de 8 à 24 atomes de carbone, dans lesquels jusqu'à trois atomes d'hydrogène aliphatiques peuvent être substitués par des groupes hydroxy, et n représente un nombre valant de 2 à 8.

**2.** Préparations photoprotectrices selon la revendication 1, **caractérisées en ce que** la ou les substance(s) filtrante(s) photoprotectrice(s) présente(s) sous forme de solide dans des conditions normales est (sont) choisie(s) parmi le groupe constitué du 4,4',4''-(1,3,5-triazine-2,4,6-triyltriimino)trisbenzoate de tris(2-éthylhexyle), du 4-méthylbenzylidènecamphre, du 4-(tert-butyl)-4'-méthoxydibenzoylméthane, de micropigments inorganiques, en particulier de micropigments inorganiques rendus hydrophobes.

**3.** Préparations photoprotectrices selon la revendication 1, **caractérisées en ce que** la ou les substance(s) filtrante(s) photoprotectrice(s) présente(s) sous forme de solide dans des conditions normales est (sont) choisie(s) parmi le groupe constitué du 4,4',4"-(1,3,5-triazine-2,4,6-triyltriimino)trisbenzoate de tris(2-éthylhexyle), du 4-méthylbenzylidènecamphre, du 4-(tert-butyl)-4'-méthoxydibenzoylméthane et du dioxyde de titane rendu hydrophobe en tant que micropigment inorganique.

**4.** Préparations photoprotectrices selon la revendication 1, **caractérisées en ce qu'** on choisit simultanément comme substances filtrantes photoprotectrices présentes sous forme de solide dans des conditions normales le 4,4', 4"-(1,3,5-triazine-2,4,6-triyltriimino)trisbenzoate de tris(2-éthylhexyle), le 4-méthylbenzylidènecamphre, le 4-(tert-butyl)-4'-méthoxydibenzoylméthane et le dioxyde de titane rendu hydrophobe en tant que micropigment inorganique.

**5.** Préparations photoprotectrices selon la revendication 1, **caractérisées en ce que** l'émulsifiant E/H choisi est le polyglycéryl-2-polyhydroxystéarate.